# EUROPEAN PATENT APPLICATION

(11) **EP 1 201 751 A1**
(43) Date of publication of application: **02.05.2002**
(21) Application number: 01309183.0
(22) Date of filing: 30.10.2001
(51) Int. Cl.: C12N 15/00, C12N 9/00, C12Q 1/68, C12N 15/11

(54) **Method for selecting highly functional nucleic acid molecules within cells**

(30) Priority: 30.10.2000 JP 2000331347
(71) Applicant: National Institute of Advanced Industrial Science and Technology, Tokyo 100-8921 (JP)
(72) Inventor: Taira, Kazunari, Tsukuba-shi, Ibaraki 305-0046 (JP); Sano, Masayuki, Tsukuba-shi, Ibaraki 305-0821 (JP)
(74) Representative: Maschio, Antonio

(57) **Abstract**

The present invention relates to a method for selecting a functional nucleic acid molecule functioning in a cell, cytoplasm or nucleus, which comprises constructing an expression vector which comprises, under the control of a promoter, a candidate nucleic acid sequence comprising a randomized sequence in a portion of a nucleic acid molecule or in another nucleic acid ligated to the nucleic acid molecule; introducing the expression vector into a cell; culturing the cell to express the nucleic acid molecule; collecting and destroying the cell to prepare an extract of an entire cell, a cytoplasm fraction or a nucleus fraction; incubating the extract of an entire cell, the cytoplasm fraction or the nucleus fraction for a certain period of time; and obtaining a nucleic acid molecule remaining in the extract of an entire cell, the cytoplasm fraction or the nucleus fraction, and to a novel functional nucleic acid molecule selected by this method.

SEQUENCE LISTING

<110> National Institute of Advanced Industrial Science and Technology

<120> Methods for selecting highly functional nucleic acid molecules within cells

<130>

<140>
<141>

<150> JP 2000-331347
<151> 2000-10-30

<160> 2

<170> PatentIn ver. 2.0

<210> 1
<211> 39
<212> DNA
<213> Artificial Sequence

<220>
<223> Description of Artificial Sequence: DNA probe

<400> 1
acatcacgtt tcggcctttc ggcctcatca gcgcggaat 39

<210> 2
<211> 137
<212> RNA
<213> Artificial Sequence

<220>
<223> Description of Artificial Sequence: Sequence of tRNA-Luc GUA Rz

<400> 2
accguugguu uccguagugu agugguuauc acguucgccu aacacgcgaa agguccccgg 60
uucgaaaccg ggcacuacaa aaaccaacuu uauuccgcgc ugaugaggcc gaaaggccga 120
aacgugaugu uuuuuuu 137

## Description

### FIELD OF THE INVENTION

The present invention relates to a method for selecting a nucleic acid molecule, which is highly stable and highly functional within cells, and also to a novel functional nucleic acid molecule selected by the method..

### BACKGROUND OF THE INVENTION

At the beginning of 1980, the ribozyme (ribonucleotide acid + enzyme) which is an RNA having a catalytic function, was found by the discovery of a self-splicing phenomenon in tetrahymena rRNA (K. Kruger, P. J. Grabowski, A. J. Zaug, J. Sands, D. E. Gottschling, T. R. Cech, Cell, 31, 147-157(1982)), and by the analysis of ribonuclease P which is a complex enzyme of RNA and protein (C. Guerrier-Takada, K. Gaydiner, T. Marsh, N. Pace, S. Altman, Cell, 35, 849-857(1983)). Thereafter, various ribozymes have been discovered (R. H. Symons, Trend. Biochem. Sci., 14, 445-450 (1989); R. H. Symons, Annu. Rev. Biochem., 61, 641-671 (1992); J. Bratty, P. Chartrand, G. Ferbeyre, R. Cedergren, Biochim. Biophys. Acta, 1216, 345-359(1993); Hasehoff. J and W. L. Gerlach, Nature, 334, 585-591 (1988); C. J. Hutchins, P. D. Rathjen, A. C. Forster, R. H. Symons, Nucleic Acids. Res., 14, 3627-3640 (1986)). As in the discovery of the reverse transcriptase, the intron and the RNA editing, the discovery of the ribozyme shook the idea of an established central dogma. At the same time, RNA which had not been recognized as anything more an informational intermediary, began, due to its two roles i.e. (genetic) information and (catalytic) function, to attract attention as a central molecule in the "RNA world" hypothesis, which places the RNA molecule as the tru origin of life (G. F. Joyce, Nature, 338, 217-224 (1989); N. R. Pace, T. L. Marsh, Origins of Life, 16, 97(1985); A. Lazcano, R. Guerrero, J. Oro, J. Mol. Evol., 27, 283 (1988); L. E. Orgel, Nature, 358, 203(1992); R. F. Gesteland, J. F. Atkins, The RNA World, Monograph 24, Cold Spring Harbor Laboratory Press, Plainview, New York (1993)).

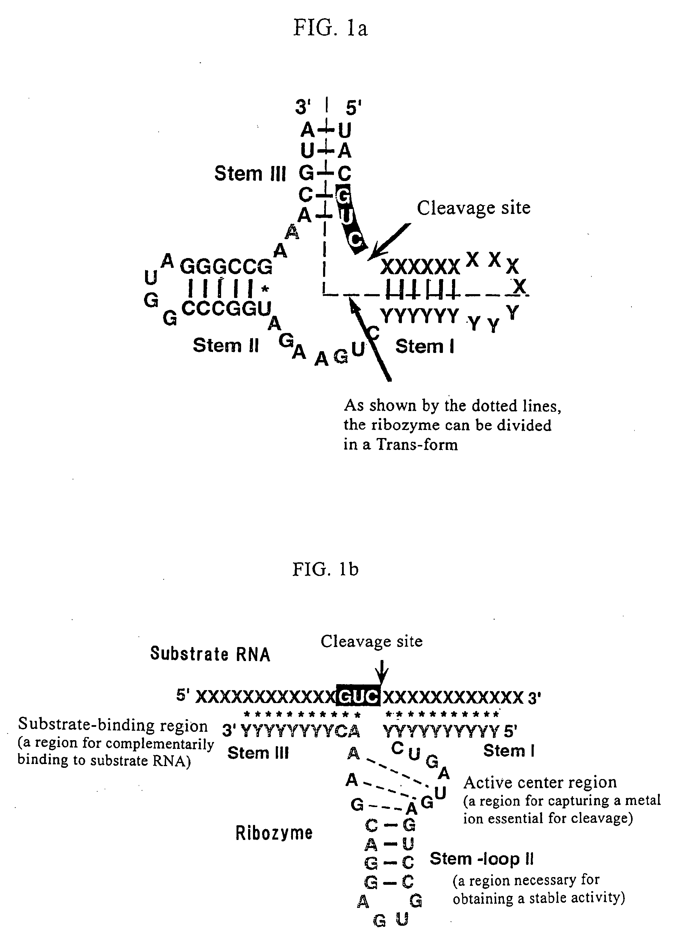

The hammerhead ribozyme is one of the most well studied ribozymes until now (Fig. 1a). This is a ribozyme which functions in nature as a self-cleaving reaction (a cis-form) (T. R. Cech, Annu. Rev. Biochem., 59, 543 (1990); A. C. Foster, R. H. Symons, Cell, 49, 211 (1987); A. C. Jeffries, R. H. Symons, Nucleic Acids Res., 17, 1371(1989)), and as the result that the ribozyme was divided into two strands of RNA (i.e., a substrate binding region and an enzyme activity retaining region) by Uhlenbeck et al. and Haseloff and Gerlach et al (O. C. Uhlenbeck, Nature, 328, 596(1987); J. Hasehoff, W. L. Gerlach, Nature, 334, 585 (1988), the possibility of applying ribozymes to gene therapy was suggested (Fig. 1b). Thereafter, a large number of applied researches which targeted cancers, AIDS, etc., have been reported (M. Cotten, M. L. Bimstiel, EMBO J8, 861 (1989); N. Sarver, E. Cantin, O. Chang, O. Lande, D. Stephens, J. Zaia, J. Rossi, Science 247, 1222 (1990); M. Homann, M. Tzortzakari, K. Rittner, S. Sczakiel, M. Tabler, Nucleic Acids Res 21, 2809 (1993); R. C. Mulligan, Science 0, 926 (1993); S. Altman: Proc. Natl. Acad. Sci. USA, 90. 10898 (1993); P. Marschall, J. B. Thompson, F. Eckstein, Cell. Mol. Neurobiol., 14, 523 (1994); S. M. Sullivan, J. Invest. Dermatol., 103, 85 (1994); F. H. Cameron, P. A. Jennings, Antisense Res. Dev., 4, 87 (1994); L. Q. Sun, D. Warrilow, L. Wang, C. Witherington, J. Macpherson, G. Symonds, Proc. Natl. Acad. Sci. USA, 91, 9715 (1994); R. E. Christoffersen, J. J. Marr, J. Med. Chem. 38, 2023 (1995); G. Ferbeyre, J. Bratty, H. Chen, R. Cedergern, Gene 155, 45(1995) ; M.Kiehntopf, E.L.Eaquivel, M.A.Brach, F.Herrmann, J.Mol. Med., 73, 65(1995); J. D. Thompson, D. Macejak, L. Couture, D. T. Stinchcomb, Nat. Med. 1, 277 (1995); T. Tuschl, J. B. Thomson, F. Eckstein, Curr. Opin. Struct. Biol. 5, 296 (1995)).

A ribozyme binds to a substrate RNA by forming a complementary base pair with the substrate. Thereafter, in the presence of a magnesium ion which is essential for the reaction, cleavage of the substrate RNA molecule occurs. As a substrate is recognized by a ribozyme via formation of appropriate base pairs between substrate-binding regions of Stem I and Stem III of the ribozyme and substrate sequences corresponding to them, the substrate specificity of the ribozyme is very high. With very high substrate specificity, any side effect hardly occurs in a cell, and consequently this will provide a considerable benefit when ribozyme is used as a gene expression inhibitor. However, it turns out that the ribozyme does not necessarily effectively function in a cell. The reasons for this are considered to be the reduction of stability or activity of a ribozyme itself in a cell as well as problems regarding expression efficiency or localization thereof. Since various types of proteins interact with a ribozyme or target RNA in a cell, RNA cleavage reaction of a ribozyme is inhibited. Further, it is also very possible that the presence of ribonuclease exerts an influence upon stability and activity of a ribozyme. To overcome such problems, stabilization of a ribozyme by chemical modification was initially proposed, but in terms of cytotoxicity, this means was not so effective (F. Eckstein, D. M. J. Lilley (eds.), Nucleic Acids and Molecular Biology, vol. 10 (1996); L. Beigelman, J. A. McSwiggen, K.G. Draper, C. Gonzalez, K. Jensen, A. M. Karpeosky, A. S. Modak, J. Matulic-Adamic, A. B. DiRenzo, P. Haeberli, D. Sweedler, D. Tracz, S. Grimm, F. E. Wincott, V. G. Thackray, N. J. Usman, B. Biol. Chem. 270, 25702 (1995)).

At present, there is mainly applied a method in which a gene encoding a ribozyme is introduced in the form of a plasmid and transcribed in a cell, and since a ribozyme can be introduced into a cell in the state of a stable DNA molecule by this method, continuous production of ribozymes can be expected using a transcription system in a cell. Moreover, since no chemical modification or analogous means is required, there is almost no toxicity to a cell.

To produce an intracellularly effective ribozyme, however, it is required to develop an expression system which is excellent in respect of the following 4 properties of a ribozyme: (1) transcriptional level, (2) intracellular stability, (3) intracellular localization, and (4) activity of the ribozyme itself. As a ribozyme expression system, the polymerase II (pol II) system, which is an mRNA transcriptional system, has previously often been used. However, the present inventors have adopted the tRNA^{val} promoter for polymerase III (pol III) system in expression of ribozymes. The transcriptional level of a ribozyme in this system is 2 or 3 orders of magnitude greater than that in pol II (M. Cotton, M. L. Birnstiel, EMBO J., 8, 3861 (1989)), and in the pol III system, a redundant additional sequence(s) does not readily attach to a ribozyme. In this case, ribozyme is transcribed in a form where it is ligated to 3' of tRNA via a linker. This linker, which ligates between tRNA and a ribozyme, has a great influence not only on the higher order structure of a ribozyme but also on the stability thereof (S. Koseki, T. Tanabe, K. Tani, S. Asano, T. Shioda, T. Shimada, Y. Nagai, J. Ohkawa, K. Taira, J. Virol., 73, 1868 (1999)). However, the optimal length and sequence of a linker are completely unknown, and consequently how to select a linker sequence still depends on trial and error, and intuition.

The intracellular localization of a ribozyme expressed by the tRNA transcriptional system varies depending on differences in higher order structure of the ribozyme. After an mRNA, which is a target of a ribozyme, is transcribed as a precursor comprising an intron, it is matured by splicing and immediately transporting to the cytoplasm. It is thought that spliceosomes or many RNA-binding proteins adhere to an immature mRNA in the nucleus, and so it is considerably difficult that ribozyme targets an mRNA in the nucleus (S. Koseki, T. Tanabe, K. Tani, S. Asano, T. Shioda, T. Shimada, Y. Nagai, J. Ohkawa, K. Taira, J. Virol., 73, 1868 (1999)). Therefore, it is more effective for a ribozyme to target an mRNA existing in cytoplasm, and if it is possible that a transcribed ribozyme moves into cytoplasm and coexists with a target mRNA therein, a higher effect can be expected. It was found that, among ribozymes expressed by the tRNA transcription system, only a ribozyme having a clover leaf structure, whose secondary structure of the first half of tRNA is similar, although not totally identical, to the original structure of tRNA, moves efficiently to cytoplasm, while a ribozyme whose structure is completely destroyed is accumulated in the nucleus (S. Koseki, T. Tanabe, K. Tani, S. Asano, T. Shioda, T. Shimada, Y. Nagai, J. Ohkawa, K. Taira, J. Virol., 73, 1868 (1999)). It has been confirmed that all the ribozymes having a structure similar to tRNA ever produced move into the cytoplasm and show effective activity (H. Kawasaki et al., Nature, 393, 284 (1998); T. Kuwabara et al., Nature Biotechnol., 16, 961 (1998); T. Kuwabara et al., Mol. Cell, 2, 617 (1998); T. Kuwabara et al., Proc. Natl. Acad. Sci. USA, 96, 1886 (1999)).

The higher order structure of a ribozyme ligated to tRNA is regulated by the sequence and length of a linker, but no tertiary structure thereof has been analyzed yet, and no mechanism regarding the transportation of the ribozyme into the cytoplasm has been found either. Thus, there has not yet been obtained a ribozyme ligated to tRNA, the higher order structure of which is regulated by an optimal linker, and which is far efficiently transported to the cytoplasm.

### SUMMARY OF THE INVENTION

Under such circumstances, the present inventors have attempted to construct a system for screening a ribozyme which has an optimal linker sequence and excellent stability and localization, from any cell.

The object of the present invention is to provide a method for selecting a functional nucleic acid molecule which is stable within cells.

The present invention is summarized below.
(1) A method for selecting a functional nucleic acid molecule functioning in a cell, cytoplasm or nucleus, which comprises: constructing an expression vector which comprises, under the control of a promoter, a candidate nucleic acid sequence comprising a randomized sequence in a portion of a nucleic acid molecule or in another nucleic acid ligated to the nucleic acid molecule; introducing the expression vector into a cell; culturing the cell to express the nucleic acid molecule; collecting and destroying the cell to prepare an extract of an entire cell, a cytoplasm fraction or a nucleus fraction; incubating the extract of an entire cell, the cytoplasm fraction or the nucleus fraction for a certain period of time; and obtaining a nucleic acid molecule remaining in the extract of an entire cell, the cytoplasm fraction or the nucleus fraction.
(2) The method of (1) above, which further comprises repeating the same cycle one or more times for the obtained functional nucleic acid molecule, provided that the period of time for incubating the extract of an entire cell, the cytoplasm fraction or the nucleus fraction is longer than that in an immediately preceding cycle.
(3) The method of (1) or (2) above, wherein the nucleic acid molecule is determined for its stability or transcriptional level, using an increase in amount of the existing nucleic acid molecule as an indicator.
(4) The method of (1) or (2) above, wherein the nucleic acid molecule is determined for its function, using activity of the nucleic acid molecule as an indicator.
(5) The method of (1) or (2) above, wherein intracellular localization of the nucleic acid molecule is determined, using the existence of the nucleic acid molecule in the cytoplasm fraction or the nucleus fraction as an indicator.
(6) The method of (1) or (2) above, wherein a linker exists in between the promoter and the nucleic acid molecule, and the linker is randomized.
(7) The method of any one of (1) to (6) above, wherein the nucleic acid molecule is selected from the group consisting of an antisense RNA, an antisense DNA, an aptamer and a DNA-RNA hybrid.
(8) The method of any one of (1) to (6) above, wherein the nucleic acid molecule is a nucleic acid enzyme.
(9) The method of (8) above, wherein the nucleic acid enzyme is a ribozyme.
(10) The method of (9) above, wherein the ribozyme is a hammerhead ribozyme.
(11) A novel functional nucleic acid molecule selected by the method according to any one of (1) to (10) above, wherein the functional nucleic acid molecule has an increased transcriptional level, stability or activity within cells or exhibits altered intracellular localization , when compared to a corresponding control nucleic acid molecule
(12) The functional nucleic acid molecule of (11) above, which is a ribozyme.
(13) The functional nucleic acid molecule of (12) above, wherein the ribozyme is located in cytoplasm and has high stability and high activity.

The terms used herein are defined as follows.

The term "functional nucleic acid" as used herein means a nucleic acid with any biological function found *in vivo* or within cells, such as an enzyme function, a catalytic function, or a function of biological inhibition or promotion.

The term "nucleic acid enzyme" as used herein means a nucleic acid with enzyme activity, such as a ribozyme, and a DNA enzyme and a DNA which expresses an enzyme activity by binding to a metal.

The term "ribozyme" as used herein refers to an RNA having a catalytic function. The term "catalytic function" refers to a function of specifically cleaving a specific site of RNA.

The term "hammerhead ribozyme" as used herein refers to a ribozyme, which binds to an RNA (especially an mRNA) as a substrate by forming a complementary base pair between the ribozyme and the RNA, and which cleaves the phosphodiester bond at the 3' of an NUH sequence wherein N represents A, G, C and U, and H represents A, C and U, and wherein any combination may be applied, but GUC can be cleaved most frequently (Figure 1).

The term "antisense RNA" or "antisense DNA" as used herein refers to a nucleic acid capable of complementarily binding to a target RNA (especially mRNA) or DNA to inhibit a function thereof. Where the target is an mRNA, the antisense RNA binds to the mRNA and inhibits the translation of the mRNA into a protein.

The term "aptamer" as used herein refers to an RNA molecule capable of binding to a protein with high affinity. It is considered that an aptamer specifically acting on a pathogenic protein can inhibit the function of the protein in a cell.

The term "polymerase III" (also referred to as "pol III") as used herein refers to a promoter suitable for expression of short RNA molecules such as ribozymes, and examples of such a promoter include a tRNA promoter, a retrovirus pol III promoter, an adenovirus VA1 promoter, etc.

The term "tRNA^{val} promoter" (also referred to as "tRNA^{val}") as used herein means one of pol III promoters, which is involved in the transcription of a short RNA molecule such as tRNA.

The term "terminator" as used herein refers to a gene of an mRNA transcription termination portion.

The term "randomized or "randomization" as used herein means preparing a pool in which any possible set of nucleotides, which are selected from A, T (optionally U), G and C, has been introduced as the nucleotides of a linker sequence.

### BRIEF DESCRIPTION OF THE DRAWINGS

Figure 1 shows the division of a ribozyme into its substrate region and its enzyme activity-retaining region (Fig. 1a), and the structure of a ribozyme and the cleavage site of a substrate (Fig. 1b).

Figure 2 shows the structure of tRNA^{val}-randomized linker sequence-ribozyme (Fig. 2a), and the construction of an expression vector comprising the structure (Fig. 2b).

Figure 3 shows a procedure for selecting a highly stable ribozyme. The structure of a control, tRNA-Luc GUA Rz, is the same as in Fig. 2a (SEQ ID NO: 2).

Figure 4 shows the results obtained by examining the stability of a ribozyme using an amount of the existing ribozyme as an indicator, by means of hybridization. The greater the number of cycles (i.e., in the order of G-1, G-2 and G-3), the greater the increase in amount of the existing ribozyme, wherein the amount is a relative value as determined when that in the first pool is 1.

Figure 5 shows the cleavage activity of a ribozyme towards luciferase mRNA as a substrate. In this figure, pUC-dt represents an expression vector which contains no ribozyme sequence, tRNA-Luc Rz (Fig. 2a) represents a conventional ribozyme (as a control), and both G-1 and G-2 represent ribozymes obtained by the first cycle and the second cycle, respectively.

### DETAILED DESCRIPTION OF THE INVENTION

The present invention provides a method for selecting a nucleic acid which is highly stable and functional in a cell or in the cytoplasm or nucleus thereof. According to one embodiment of the present invention, there is provided a method for selecting, under a certain selection pressure, a highly stable and active ribozyme from a pool of tRNA ribozyme-expression plasmids, in which a linker portion, through which tRNA promoter and ribozyme are ligated, is randomized.

The nucleic acid molecule selected by the method of the present invention is characterized in that it is highly functional. The term "highly functional" as used herein means that the nucleic acid molecule is excellent in total in respect of properties such as transcriptional level, intracellular stability, intracellular localization and activity. Various types of highly functional nucleic acids can be selected by altering the type and strength of selection pressure. The reason why the function of a functional nucleic acid varies in a cell is likely to be based upon the fact that the nucleic acid may have various types of higher order structures depending on their sequences and thus the nucleic acid-recognizing patterns of various proteins are altered. For example, a nucleic acid which is easily recognized by a nuclease is unstable in a cell; and a nucleic acid recognized by a transport receptor is localized at an appropriate site.

By examining the nucleotide sequence of a highly functional nucleic acid selected by the present invention, the sequence and characteristics of a higher order structure necessary for efficient function of the nucleic acid within cells can be clarified. Such information is not only useful for development of a highly efficient nucleic acid-expression system directed to current or future applications in individuals, but also useful for the analysis of any protein, pathway or mechanism which determines the fate of a nucleic acid within cells.

A method for selecting a nucleic acid molecule highly functional within cells will be described hereafter.

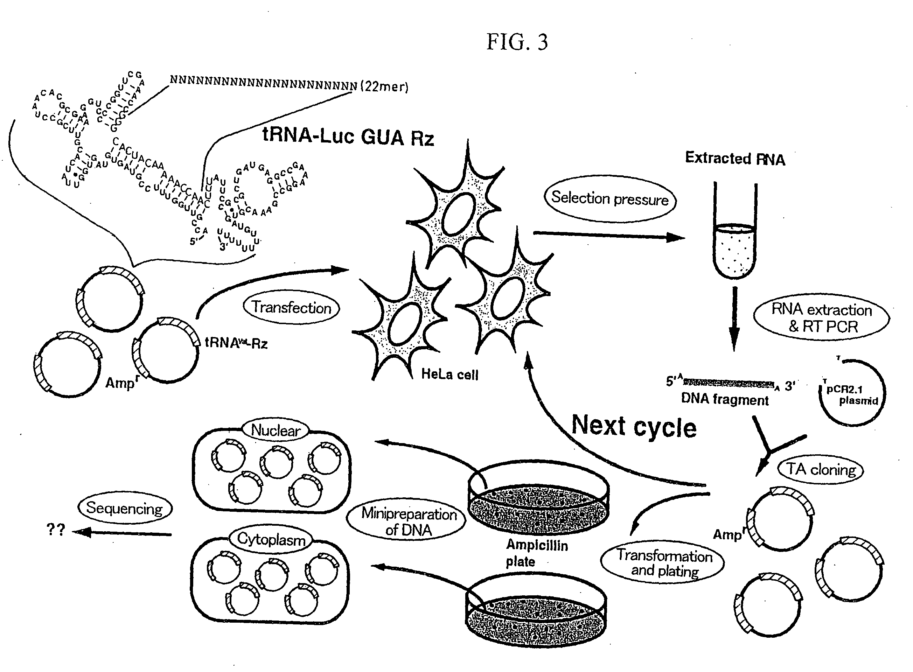

According to the present invention, the method comprises constructing an expression vector which comprises, under the control of a promoter, a candidate nucleic acid sequence comprising a randomized sequence in a portion of a nucleic acid molecule or in another nucleic acid ligated to the nucleic acid molecule; introducing the expression vector into a cell; culturing the cell to express the nucleic acid molecule; collecting and destroying the cell to prepare an extract of an entire cell, a cytoplasm fraction or a nucleus fraction; incubating the extract of an entire cell, the cytoplasm fraction or the nucleus fraction for a given period of time; and obtaining a nucleic acid molecule remaining in the extract of an entire cell, the cytoplasm fraction or the nucleus fraction (Figure 3).

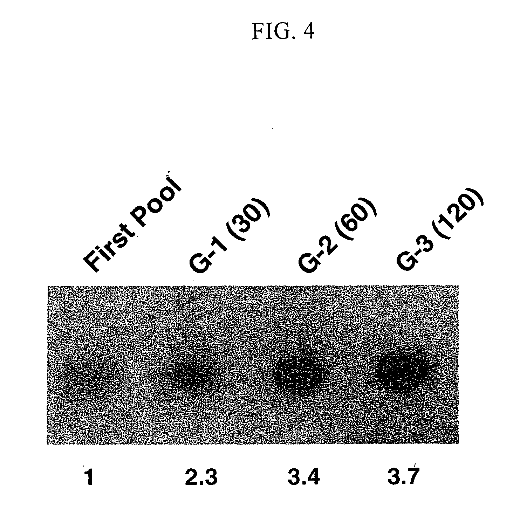

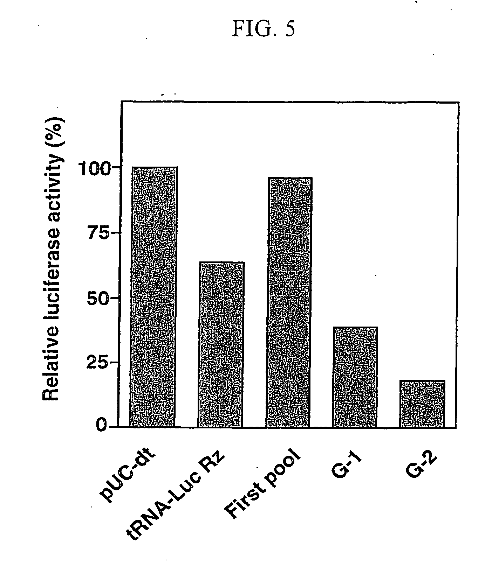

A feature of the present invention is that a structurally changeable portion is randomized in a portion of a nucleic acid molecule or in another nucleic acid (particularly, a single-stranded RNA or DNA) ligated to the nucleic acid molecule, and that *in vivo* screening is carried out based on selection pressures such as stability, transcriptional level, intracellular localization and activity, using any cell. Furthermore, the same cycle as described above is repeated for the obtained desired nucleic acid molecule one or more times, but where the period for incubating the extract of an entire cell, the cytoplasm fraction or the nucleus fraction is set to be longer than that in the immediately preceding cycle, a nucleic acid which is more stable thus more highly active, can be obtained (Figs. 4 and 5).

The type of the target nucleic acid molecule of the present invention is not particularly limited. Examples of the target nucleic acid molecule include a nucleic acid enzyme (e.g. a ribozyme, a DNA enzyme, a DNA which expresses an enzyme activity by binding to a metal), an antisense RNA, an antisense DNA, an aptamer, a DNA-RNA hybrid, etc. The preferable nucleic acid molecule is a ribozyme, particularly a hammerhead ribozyme (Fig. 1).

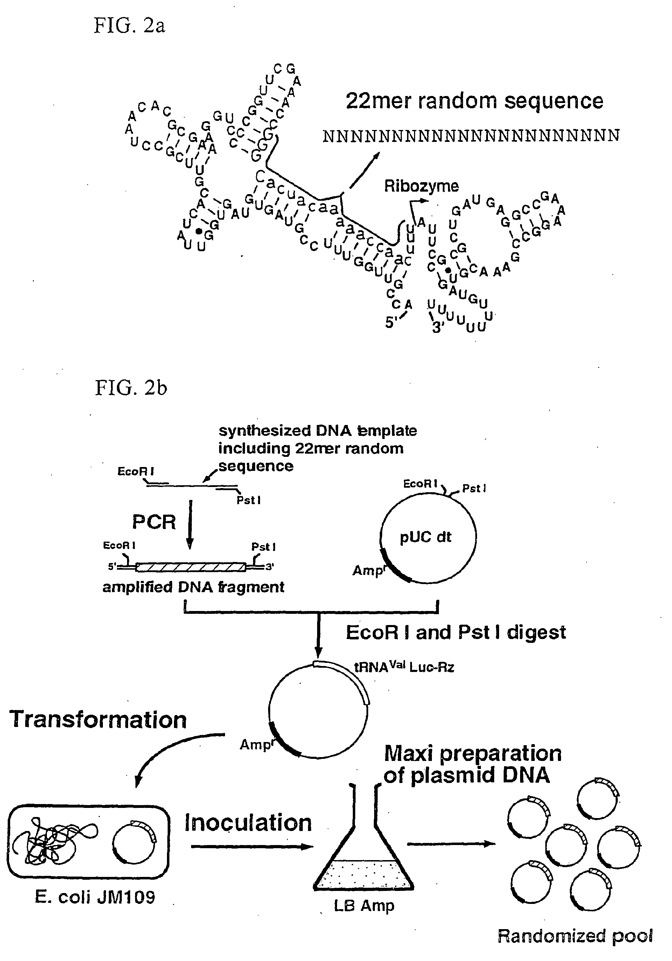

Examples of randomization include the randomization of a linker sequence existing in between a nucleic acid molecule-encoding sequence and a promoter sequence (Fig. 2a). The length of a linker sequence is, but is not limited to, one or more bases, normally 1 to 1,000 bases, preferably 10 to 100 bases.

The expression vector comprises a promoter which allows a nucleic acid molecule to express in a cell. The promoter is not particularly limited, but where the nucleic acid molecule is a ribozyme, examples of an applicable promoter include a polymerase II promoter and a polymerase III promoter, preferably a polymerase III promoter, such as a tRNA promoter, a retrovirus pol III promoter and an adenovirus VA1 promoter. Of them, a tRNA promoter is preferable, and a tRNA^{val} promoter (also referred to as "tRNA^{val}") or a variant thereof is more preferable. An example of an expression vector comprising tRNA^{val} is a pUC dt plasmid, which comprises unique EcoR I and Pst I sites and an ampicillin-resistant gene (Amp^{r}) (Koseki, S. et al., J. Virol. 73, 1868-1877 (1999)). Other examples of a vector to construct the expression system include pUC19 (Takara Shuzo), pGREEN LANTERN (Lifetech Oriental), pHaMDR (Human Gene Therapy 6, 905-915 (1995)), an adenovirus vector and a retrovirus vector. The vectors can comprise not only a gene of interest and a promoter, but also elements such as a replication origin, a terminator (i.e., a transcription termination sequence), and a selective marker gene (e.g. an antibiotic-resistant gene or an auxotrophy-complement gene)

To introduce a vector into a cell, common methods can be used. For example, they include the calcium phosphate method, the electroporation method, the lipofection method, the microinjection method, and the liposome method.

PCR amplification is carried out using, as a template, a synthesized DNA comprising a randomized sequence in order to obtain an amplified DNA fragment (using conditions as described by Sambrook, J. et al., In "Molecular Cloning A Laboratory Manual", Cold Spring Harbor Laboratory Press (1989)). In this case, primers may be designed so that a desired restriction enzyme site(s) (which is preferably identical to the unique restriction site of an expression vector) is formed at each terminus of the fragments. The resulting DNA fragments are incorporated into an expression vector treated with the same restriction enzyme(s), and a bacterial host such as *Escherichia coli* is transformed with this vector. Then, the obtained transformant is incubated (or cultured) in an appropriate medium, and antibiotic-resistant clones are selected to be used as a randomized pool (Fig. 2b).

Subsequently, using the randomized pool, a mammalian cell (e.g. HeLa cell, Xenopus oocyte, etc.) is transfected, and is then subjected to selection pressure. That is, after culture, the cell is destroyed by a mechanical (e.g. homogenization) or chemical (e.g. hypo-osmosis and lysozyme) method to prepare a cell extract, cytoplasm fraction or nucleus fraction, which is then incubated for a given period of time (e.g. 30 minutes, 60 minutes or 120 minutes). As a result, a nucleic acid molecule with high stability remains. Then, this molecule is collected. In this method, the amount (or transcriptional level) of the existing nucleic acid molecule can be determined from the degree of hybridization (e.g. spot size or density) by hybridizing the nucleic acid molecule with a DNA probe which has a complementary sequence to the nucleic acid molecule. Where the nucleic acid molecule is a nucleic acid enzyme, the degree of its function can be confirmed by determination of the enzym activity. The enzyme activity of a ribozyme can be evaluated by determining the cleavage activity using an activity of a substrate RNA as an indicator (Kuwabara, T. et al., Proc. Natl. Acad. Sci. USA 96, 1886-1891 (1999)). That is, as the activity of a substrate RNA is lower, the cleavage activity is higher. The localization of a nucleic acid molecule can be determined by examining the existence of the nucleic acid molecule in the cytoplasm or nucleus fraction (e.g. by hybridization method).

In the method of the present invention, the above-described cycle, which comprises the incorporation of a nucleic acid molecule into a vector, the transfection of a mammalian cell with the vector, and the preparation and incubation of the extract of an entire cell, the cytoplasm fraction and the nucleus fraction, can be repeated one or more times for the obtained functional nucleic acid. It is more likely that stable and functional nucleic acid molecules can be obtained by repeating the cycle (Figs. 4 and 5). In this case, it is better that the incubation period takes longer than that in the immediately preceding cycle. For example, if the incubation period in the previous cycle is 30 minutes, it can be 60 minutes in the present cycle, and 120 minutes in the next cycle.

Intracellular stability has previously been studied, and it has been confirmed that an intracellularly stable ribozyme has a higher activity than unstable one (S. Koseki et al., J. Virol., 73, 1868 (1999)). The early clarification of the correlation between the higher order structure of a ribozyme and the stability thereof has been desired in order to design a highly functional ribozyme, but it is still completely unknown. However, the correlation between the higher order structure and the stability can be clarified by prediction of the secondary structure of a highly stable ribozyme using the Zuker method (Zuker, M. et al., Nucleic Acids Res. 9, 133-148 (1981)), the ribozyme being screened within a cell by the method of the present invention. According to this method, a stable ribozyme having high activity can easily be designed by the prediction of secondary structure.

With regard to the intracellular localization of a ribozyme, more efficiently transported ribozyme can be selected by screening within a cell. For example, a ribozyme which is more efficiently transported to the cytoplasm can be selected by adding an excess amount of a plasmid which encodes RNA competing with transportation of a ribozyme, and saturating the transportation pathway of a ribozyme.

Since the screening can be carried out *in vivo* in the present invention, it is ensured that the actually selected ribozyme acts effectively in a cell. Furthermore, since there have been almost no examples regarding that how to ligate an expression system with a ribozyme has been focused on and studied, and in particular, this study directed to the optimization of a linker is the first attempt. If expression system for more highly functional ribozymes can be designed with the characteristics of a ribozyme obtained by the screening, and it is clarified that the ribozyme has a higher activity than the conventional expression systems, it can be applied for an individual.

The present invention further provides a novel nucleic acid molecule, which is highly stable and functional within cells and is selected by the above method. An example of the nucleic acid molecule is a ribozyme. The present invention is characterized in that the intracellular transcriptional level, stability or activity of the nucleic acid molecule increases, or the intracellular localization thereof is altered in comparison with a corresponding control nucleic acid molecule. An example of such a molecule is a ribozyme which is located in cytoplasm and has high stability and high activity. Any novel nucleic acid molecule having the above characteristics, which can be selected by the method of the present invention, is included in the present invention.

### EXAMPLES

The present invention will be more specifically described in the following examples. The examples are not intended to limit the scope of the invention.

### Example 1. Construction of initial pool and screening of ribozyme with good stability

According to the known method (Kawasaki, H. et al., Nature 393, 284-289 (1998); Kuwabara, T. et al., Mol. Cell 2, 617:627 (1998)), expression vectors were constructed by inserting a ribozyme sequence at a site downstream of a tRNA^{val} promoter via a linker with randomized sequence (Figs. 2a and 2b). This initial (or first) pool contained a large number of ribozyme expression vectors which comprise linkers with different nucleotide sequences.

An aliquot of the expression vectors was transfected to HeLa S3 cells, using a transfection reagent (Trans 1T-LT1 reagent; Pan Vera, Madison, WI, USA). After 24 hours, the cells were collected and an extract of the cells was prepared. The obtained extract was kept at 37°C for a certain period of time, to select ribozymes having high stability by using resistance to nucleases in a cell as an indicator.

After purification of ribozymes, double stranded DNAs encoding ribozyme sequences which were selected by RT-PCR were obtained. These obtained DNAs were inserted into plasmids to prepare ribozyme expression vectors. After cloning, a portion of ribozymes was subjected to sequencing to confirm the sequence of a randomized portion. A series of procedures are shown in Fig. 3.

After transfecting the obtained expression vectors into HeLa S3 cells again, the collection of cells and preparation of an extract were carried out in the same procedures as described above. The extract was kept at 37°C for twice as long as the above time in order to select more stable ribozymes. After purification of ribozymes, double stranded DNAs were obtained by RT-PCR, and the DNAs were inserted into plasmids to prepare ribozyme expression vectors again.

In the next cycle, the incubation period was extended to twice as long as the time in the previous cycle (4 times the period in the first cycle), and the same procedures were performed.

### Example 2. Stability of selected ribozyme

The stability of the selected ribozymes was evaluated as follows. An aliquot of expression plasmids in a bulk (in which ribozyme expression vectors were mixed), which encode ribozymes (generations 1 to 3, or G-1 to G-3) selected in each cycle, was transfected into HeLa S3 cells, and after about 24 hours, the existing amount of ribozymes was determined by Northern hybridization. Because the more stable the ribozyme is in a cell, the longer the time required for decomposition of a ribozyme by nucleases, the existing amount of a stable ribozyme is likely to be larger. RNA extracted from cells was subjected to agarose gel electrophoresis and transferred to a nucleic acid adsorption membrane. Then, a DNA probe (5'-aca tca cgt ttc ggc ctt tcg gcc tca tca gcg cgg aat-3'; SEQ ID NO: 1) having a sequence complementary to the 5'-labeled ribozyme was hybridized on the membrane (hybridization conditions: Ambion ULTRAhyb buffer, 42°C, overnight). After that, the existing ribozyme was detected using STORM830 image analyzer (Molecular Dynamics). As a result, it was found that the existing amount of the ribozyme increased as generations proceeded (Fig. 4). This is because more stable ribozyme was selected by each turnover of the selection cycle. By this method, it was confirmed that the present invention effectively works in selection of an highly functional nucleic acid within cells.

### Example 3. Target RNA-cleavage activity of selected ribozyme in cell

Since the ribozyme used in the present invention targets a luciferase gene, whether the increase of stability raises the activity of a ribozyme was confirmed by examining the luciferase mRNA- cleavage activity of the ribozyme obtained in each generation (Kuwabara, T. et al., Proc. Natl. Acad. Sci. USA 96, 1886-1891 (1999)). First, the ribozyme expression plasmids with high stability obtained in the first pool, G-1 and G-2 were transfected in a bulk state into the transformed cell derived from HeLa S3 cells, having a luciferase gene incorporated into its genome. After a predertermined period of time for culture, the effect of a ribozyme was evaluated using luciferase activity in an extract of the cell as an indicator. As a control, the activity of a tRNA ribozyme targeting a luciferase gene was also evaluated. As a result, it was found that the luciferase mRNA cleavage effect of a ribozyme became remarkable as generations proceeded and showed an inhibition effect of about 80% in generation 2 (Fig. 5). From this result, it was confirmed that the present invention was excellent in stability and was effective for selection of a ribozyme highly active in a cell.

### Industrial Applicability of the Invention

The method of the present invention can be used for selection of a nucleic acid molecule with high activity, which functions effectively in a cell. Furthermore, a ribozyme expression system with high efficiency can be developed based on a linker sequence which a highly functional ribozyme has, and this expression system can be applied for treatment of malignant diseases such as cancers and infectious diseases including AIDS. The present invention is effective for selecting not only ribozymes but also various types of functional nucleic acids, and enables a wide range of applications by altering types of nucleic acids to be used and/or types of selection pressure.

## Claims

1. A method for selecting a functional nucleic acid molecule functioning in a cell, cytoplasm or nucleus, which comprises: constructing an expression vector which comprises, under the control of a promoter, a candidate nucleic acid sequence comprising a randomized sequence in a portion of a nucleic acid molecule or in another nucleic acid ligated to said nucleic acid molecule; introducing said expression vector into a cell; culturing said cell to express said nucleic acid molecule; collecting and destroying said cell to prepare an extract of an entire cell, a cytoplasm fraction or a nucleus fraction; incubating said extract of an entire cell, said cytoplasm fraction or said nucleus fraction for a certain period of time; and obtaining a nucleic acid molecule remaining in said extract of an entire cell, said cytoplasm fraction or said nucleus fraction.

2. The method according to claim 1, which further comprises repeating the same cycle one or more times for the obtained functional nucleic acid molecule, provided that the period of time for incubating said extract of an entire cell, said cytoplasm fraction or said nucleus fraction is longer than that in an immediately preceding cycle.

3. The method according to claim 1, wherein the nucleic acid molecule is determined for its stability or transcriptional level, using an increase in amount of the existing nucleic acid molecule as an indicator.

4. The method according to claim 1, wherein the nucleic acid molecule is determined for its function, using activity of the nucleic acid molecule as an indicator.

5. The method according to claim 1, wherein the nucleic acid molecule is determined for its intracellular localization, using the existence of the nucleic acid molecule in said cytoplasm fraction or said nucleus fraction as an indicator.

6. The method according to claim 1, wherein a linker exists in between the promoter and the nucleic acid molecule, and the linker is randomized.

7. The method according to claim 1, wherein the nucleic acid molecule is selected from the group consisting of an antisense RNA, an antisense DNA, an aptamer and a DNA-RNA hybrid.

8. The method according to claim 1, wherein the nucleic acid molecule is a nucleic acid enzyme.

9. The method according to claim 8, wherein the nucleic acid enzyme is a ribozyme.

10. The method according to claim 9, wherein the ribozyme is a hammerhead ribozyme.

11. A novel functional nucleic acid molecule selected by the method according to claim 1, wherein the functional nucleic acid molecule has an increase transcriptional level, stability or activity within cells or exhibits altered intracellular localization, when compared to a corresponding control nucleic acid molecule.

12. The functional nucleic acid molecule according to claim 11, which is a ribozyme.

13. The functional nucleic acid molecule according to claim 12, wherein said ribozyme is located in cytoplasm and has high stability and high activity.
